# EUROPEAN PATENT APPLICATION

(11) **EP 3 329 907 A1**
(43) Date of publication of application: **06.06.2018**
(21) Application number: 16833233.6
(22) Date of filing: 25.07.2016
(51) Int. Cl.: A61K 9/14, A61K 9/00, A61K 47/26, A61K 31/41

(54) **DEFERASIROX-CONTAINING POWDER AND METHOD FOR PREPARING SAME**

(30) Priority: 31.07.2015 KR 20150108860
(71) Applicant: Kuhnil Pharm. Co., Ltd., Chungcheongnam-do 31032 (KR)
(72) Inventor: KU, Jeong, Goyang-si Gyeonggi-do 10508 (KR); KIM, Eunhye, Seoul 08734 (KR)
(74) Representative: Andrews, Robert
(86) International application number: PCT/KR2016/008087
(87) International publication number: WO 2017/022997

(57) **Abstract**

Embodiments of the present invention provide powder including 20 wt% to 45 wt% of a combination of lactose and sucrose with respect to the total weight of the powder and including, as an active ingredient, deferasirox or a pharmaceutically acceptable salt thereof, and a method of preparing the same.

## Description

### TECHNICAL FIELD

The present invention relates to oral powder including deferasirox as an active ingredient and a method of preparing the same, and more particularly, to deferasirox-containing oral powder that includes a sufficient amount of an active ingredient, is highly conveniently administered, has low concern about side effects and stability, and has excellent bioavailability and excellent properties of powder particles, and a method of preparing the same.

### BACKGROUND ART

Deferasirox, which is an orally active iron chelator prescribed in the treatment of iron overload, is a drug that is used in the treatment of hemochromatosis, which is the most common type of iron overload, or is prescribed in the treatment of iron overload in transfusion-dependent anemias requiring a large number of transfusions (e.g., thalassemia major, thalassemia intermediate, and sickle cell disease).

The hemochromatosis, which is the most common form of iron overload disease, is an inherited disorder that causes the body to absorb and store too much iron. An excess of iron builds up in organs and damages them. Without treatment, the disease can cause these organs to fail.

Patients with transfusion-dependent anemias require chelation therapies to remove accumulated iron from the body due to a large number of transfusions.

The deferasirox has a structure represented by Formula 1 below, and salts thereof and crystal forms thereof are disclosed in Patent Document 1:

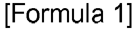

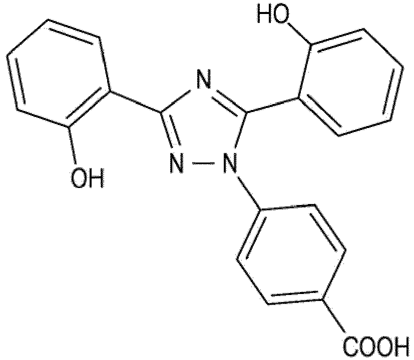

Generally, a daily dose of deferasirox prescribed for the treatment of thalassemia is large, for example, 5 mg/kg (body weight)/day to 40 mg/kg (body weight)/day for adults or children. Due to such a high dose, when a single tablet containing a daily dose is formulated, the size of tablets is too large for adults or children to swallow, thus not allowing the formulation of general tablets. To address this problem, deferasirox dispersible tablets for oral administration that contain a daily dose and are conveniently administered to adults and children have been developed (Patent Document 2). The contents of pharmaceutical additives in the dispersible tablet are very high based on the total weight of the tablet.

The dispersible tablets are currently commercially available under the product name of Exjade® dispersible tablet 125 mg, 250 mg, and 500 mg (NOVARTIS), and the total weight of a tablet containing 500 mg of deferasirox free acid is 1,700 mg, wherein the percentage of additives is very high. Although pharmaceutically acceptable additives are used, sensitive people may undergo side effects due to the additives, such as indigestion, e.g., bloated stomach, flatulence, and the like, and thus it is preferably to use additives in a small amount.

In addition, it has been verified that, when the dispersible tables are prepared into a suspension in an aqueous phase (e.g., water) for ingestion purposes, the stability of an active ingredient thereof is not good, and, when a patient ingests a suspension prepared for administration after a certain period of time, not directly ingesting the suspension, due to many different conditions, sufficient efficacy thereof cannot be obtained due to low stability of the active ingredient (see Patent Document 3). Indeed, in busy modern societies, the ingestion of a dispersible tablet after dissolution thereof is forgotten or tends to be delayed to deal with more urgent tasks.

In addition, deferasirox is highly water-insoluble, and thus, when deferasirox is orally administered, it is important to secure a rapid dissolution rate to have sufficient bioavailability.

### [Prior Art Documents]

### [Patent Documents]

(Patent Document 1) WO97/49395
(Patent Document 2) WO04/035026
(Patent Document 3) WO12/003987

### DETAILED DESCRIPTION OF THE INVENTION

### TECHNICAL PROBLEM

An object of the present invention is to provide deferasirox powder that may include a daily effective dose of deferasirox, is highly conveniently administered, has reduced concern about side effects due to additives because the percentage of additives is not high, and secures drug stability and efficacy.

Another aspect of the present invention is to provide a method of preparing the deferasirox powder.

### TECHNICAL SOLUTION

An aspect of the present invention provides
powder including 20 wt% to 45 wt% of a combination of lactose and sucrose with respect to a total weight of the powder, and containing, as an active ingredient, a compound represented by Formula 1 below or a pharmaceutically acceptable salt thereof:

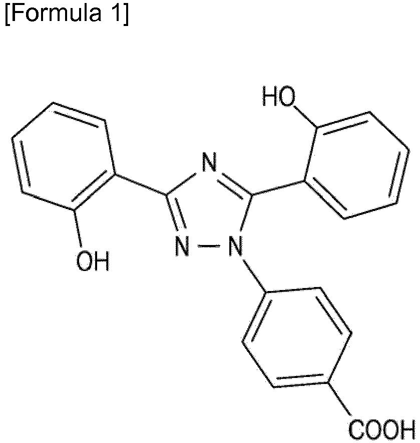

According to another aspect of the present invention provides a method of preparing the powder according to an aspect of the present invention, including:
weighing raw materials including a compound represented by Formula 1 above or a pharmaceutically acceptable salt thereof as an active ingredient, lactose, and sucrose; and
mixing, kneading, granulating, and milling the weighed raw materials.

### ADVANTAGEOUS EFFECTS OF THE INVENTION

Deferasirox powder according to the present invention may be directly administered or administered as a suspension in an aqueous phase, and thus may contain a daily dose of deferasirox that requires high dosage strength and is highly conveniently administered because there is no concern about choked throat as in tablets when swallowed. In addition, the deferasirox powder includes a lower percentage of additives than that of conventionally commercially available dispersible tablets, and thus may have reduced side effects due to the additives. Furthermore, the deferasirox powder can be directly ingested without being suspended in an aqueous phase, unlike commercially available dispersible tablets, and thus concern about the deterioration of drug stability that may occur due to the preparation of a suspension and leaving a suspension unused. In addition, the deferasirox powder according to the present invention may exhibit the same dissolution rate as that of commercially available dispersible tablets and may be prepared as powder with excellent properties.

### DESCRIPTION OF THE DRAWINGS

FIG. 1 is a graph showing comparative dissolution test results according to time of deferasirox-containing powders prepared by varying the type of filler and Exjade as a control.
FIG. 2 is a graph showing comparative dissolution test results according to time of deferasirox-containing powders prepared to contain, as a filler, combinations of lactose and sucrose at various ratios and Exjade as a control.
FIG. 3 is a graph showing comparative dissolution test results according to time of deferasirox powders prepared to contain, as a filler, combinations of lactose and microcrystalline cellulose at various ratios and Exjade as a control.
FIG. 4 is a graph showing comparative dissolution test results according to time of deferasirox powders prepared by varying the type of surfactant and Exjade as a control.
FIG. 5 is a graph showing comparative dissolution test results according to time of deferasirox powders prepared by varying the content of Tween 80 as a surfactant and Exjade as a control.
FIG. 6 is a graph showing comparative dissolution test results according to time of deferasirox powders prepared by varying the content of sodium lauryl sulfate as a surfactant and Exjade as a control.
FIG. 7 is a graph showing comparative dissolution test results according to time of deferasirox powders prepared using a high speed mixer or an extruder and Exjade as a control.

### MODE OF THE INVENTION

All technical terms used in the present invention are used in the sense that they are generally understood by those of ordinary skill in the art to which the present invention pertains unless otherwise defined. In addition, preferred methods or samples are described in the present specification, but similar or equivalent ones are also within the scope of the present invention. The content of all publications described in the present specification is incorporated herein by reference in their entirety.

An embodiment of the present invention provides
powder including 20 wt% to 45 wt% of a combination of lactose and sucrose with respect to a total weight of the powder and containing, as an active ingredient, a compound represented by Formula 1 below or a pharmaceutically acceptable salt thereof:

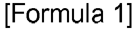

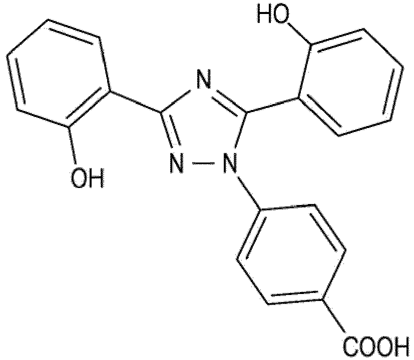

The generic name of the compound of Formula 1 is deferasirox, and the compound of Formula 1, salts thereof, and crystal forms thereof are disclosed in International Patent Application Publication No. WO97/49395 incorporated herein by reference. In one embodiment, the compound of Formula 1 has a crystal form described in Example 5 of WO97/49395.

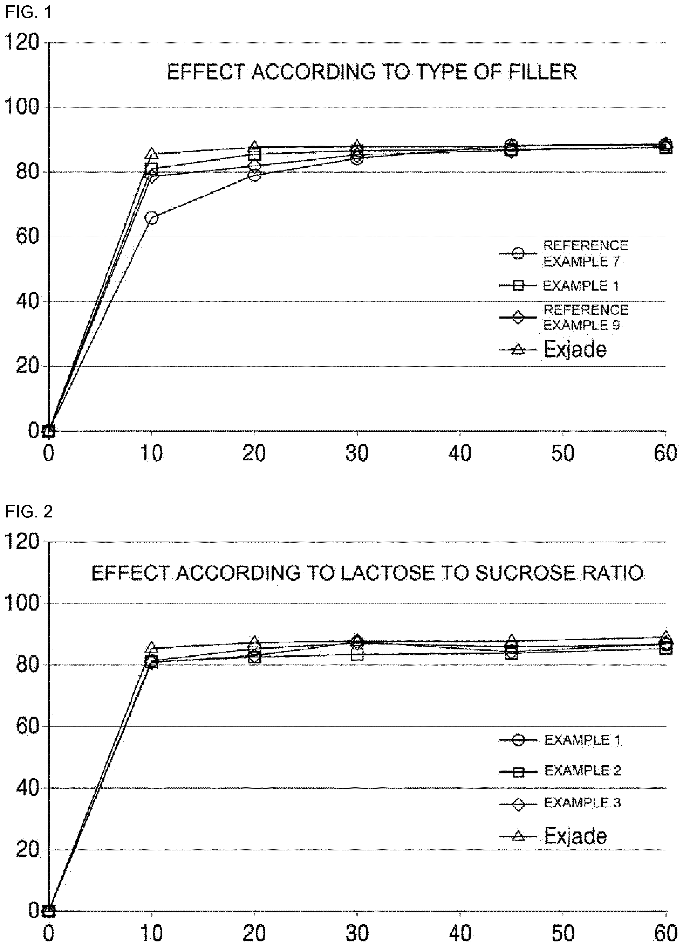

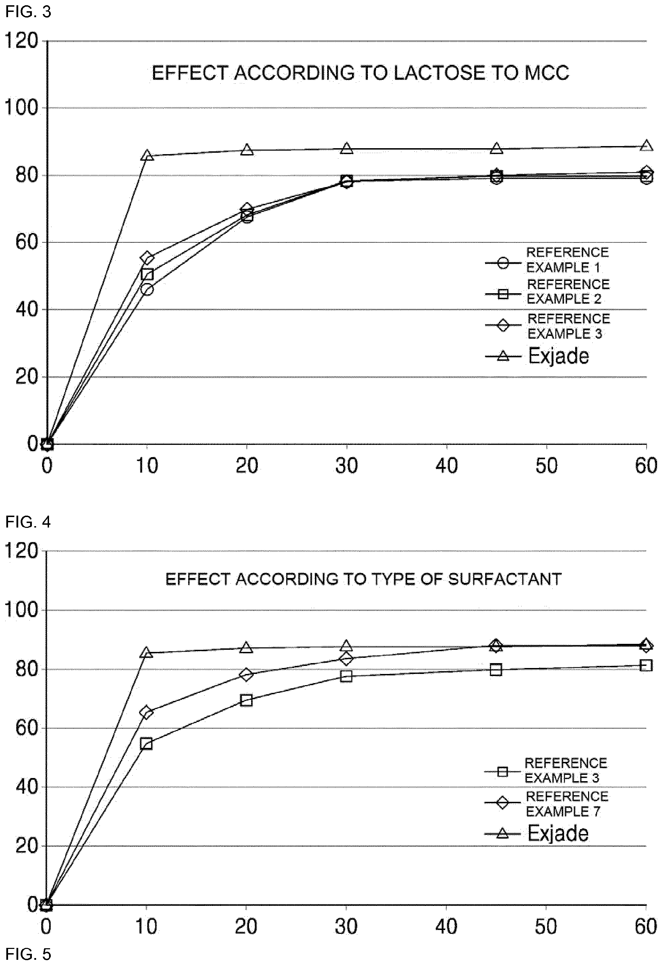

In the powder, the combination of lactose and sucrose act as a filler, and it was confirmed that, when compared to a case in which other types of fillers were used, a dissolution rate of the active ingredient was significantly high and was equivalent to that of commercially available dispersible tablets (see FIG. 1). In one embodiment, the filler may be included in an amount of about 20 wt% to about 45 wt%, more particularly, 25 wt% to 42 wt%, with respect to the total weight of the powder. In the case of including lactose and microcrystalline cellulose instead of the combination of lactose and sucrose, an increasing pattern of dissolution rate was shown as the percentage of lactose increased, but distinctly lower dissolution rate was shown compared to that of commercially available deferasirox dispersible tablets (see FIG. 3).

The combination ratio of lactose to sucrose is not particularly limited, and may range, for example, from about 1:4 to about 4:1 on a weight ratio basis. It was confirmed that, within the above ratio range, the dissolution rate of the active ingredient was hardly changed according to a change in the percentage of sucrose (see FIG. 2), but it was confirmed that, since problems, such as the generation of a large amount of fine powder and the like, occur when the percentage of sucrose is low, as the percentage of sucrose increased, properties of particles constituting the powder were enhanced. Thus, in one embodiment, the combination ratio of lactose to sucrose may range from 1:1 to 1:4, more particularly, 1:2.5 to 1:3.5 on a weight ratio basis.

The lactose is lactose, a lactose monohydrate, a lactose monohydrate (200 mesh), or spray-dried lactose.

The powder may include a pharmaceutically effective amount of an iron chelator, and may include the active ingredient in an amount of, particularly, about 35 wt% to 50 wt%, more particularly, about 35 wt% to about 42 wt%, with respect to the total weight of the powder. The powder includes a high percentage of the active ingredient with respect to a total weight of the preparation and may exhibit an equivalent dissolution rate to that of currently commercially available deferasirox dispersible tablets, thus exhibiting equivalent efficacy thereto. In addition, the powder includes a lower percentage of additives present in the preparation than that of currently commercially available deferasirox dispersible tablets (Exjade®: 30 wt% or less of an active ingredient), and thus side effects due to the additives may be reduced.

The powder may further include a known additional additive that may be commonly used in the formulation of powders, and, in particular, may further include a disintegrating agent, a binder, a surfactant, or an arbitrary combination thereof.

The disintegrating agent may be any known disintegrating agent that may be used in the formulation of powders in the art, and, in one embodiment, may be selected from the group consisting of crospovidone, croscarmellose sodium, sodium starch glycolate, low substituted hydroxypropylcellulose, and combinations thereof. The amount of the disintegrating agent may range from about 15 wt% to about 30 wt% with respect to the total weight of the powder.

The binder may be any known binder that may be used in the formulation of powders in the art, and, in one embodiment, may be selected from the group consisting of polyvinylpyrrolidone (e.g., PVP K30), hydroxypropylcellulose, hydroxypropylmethylcellulose, anhydrous silicic acid, sodium carboxymethylcellulose, polyethylene glycol 6000, polypropylene glycol, glycerin fatty acid esters, calcium carbonate, and combinations thereof. The amount of the binder may range from about 1.5 wt% to about 5 wt% with respect to the total weight of the powder.

The surfactant may be any known surfactant that may be used in the formulation of powders in the art, and, in one embodiment, may be selected from the group consisting of sodium lauryl sulfate (SLS), polyoxyethylene fatty acid esters (Tween®, e.g., Tween 80), and combinations thereof. The amount of the surfactant may range from about 0.2 wt% to about 10 wt%, particularly, about 0.2 wt% to about 1.5 wt%, and more particularly, about 0.2 wt% to about 0.5 wt%, with respect to the total weight of the powder.

The surfactant acts as a solubilizer in the powder.

In one embodiment, the surfactant is sodium lauryl sulfate.

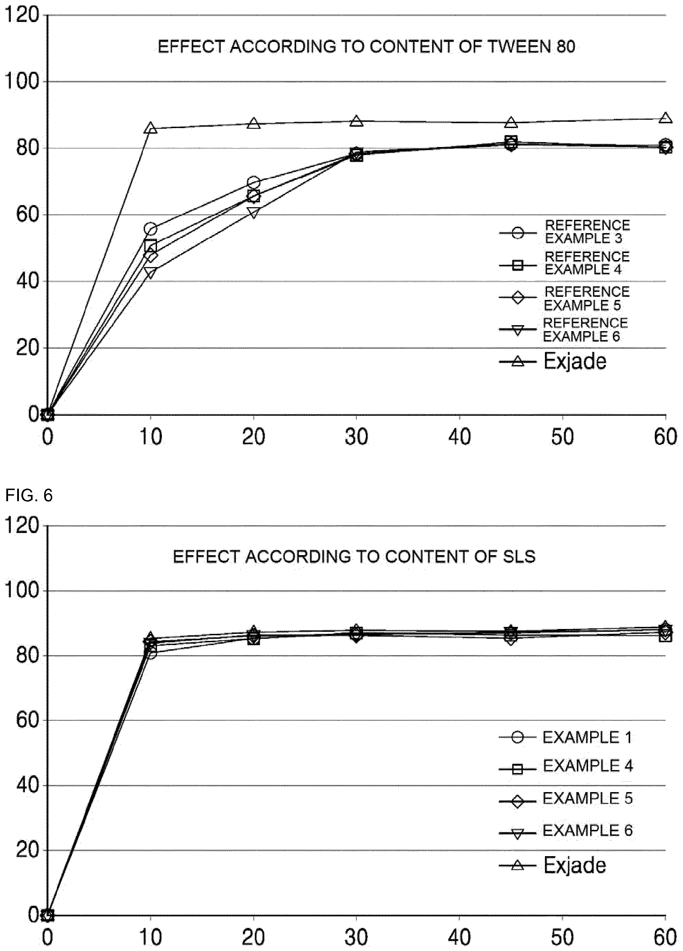

As a result of experimentation, a case in which sodium lauryl sulfate was used as a surfactant exhibited a significantly higher dissolution rate than in a case in which Tween 80 was used as a surfactant (see FIG. 4). As a result of experimentation, Tween 80 exhibited an increasing pattern of dissolution rate as the amount thereof in the powder increased, while exhibiting a distinctly lower dissolution rate than that of commercially available deferasirox dispersible tablets (see FIG. 5).

The amount of sodium lauryl sulfate may be appropriately selected by one of ordinary skill in the art from amounts that enable the sodium lauryl sulfate to function as a solubilizer, and may range from about 0.2 wt% to about 10 wt%, more particularly, 0.2 wt% to 1.5 wt%, with respect to the total weight of the powder. As a result of experimentation, the dissolution rate of the active ingredient was seen to hardly change according to a change in the amount of sodium lauryl sulfate and an equivalent dissolution rate to that of a control was shown even when 0.5 wt% of sodium lauryl sulfate was used (see FIG. 6). Since surfactants have concern about side effects, the amount of surfactant may be reduced in view of the above experimental results. In one embodiment, the sodium lauryl sulfate may be included in an amount of 0.2 wt% to 0.5 wt% with respect to the total weight of the powder. As such, due to inclusion thereof in a small amount, the powder may include about 6 mg or less of sodium lauryl sulfate with respect to 500 mg of free acid as an active ingredient, and thus the amount of the surfactant is significantly decreased with respect to the active ingredient compared to conventional commercially available deferasirox dispersible tablets (Exjade 500 mg tablet contains about 8.4 mg of SLS). Accordingly, concern about the generation of side effects due to the surfactant may be reduced.

In one embodiment, the powder includes crospovidone as a disintegrating agent, polyvinylpyrrolidone as a binder, and sodium lauryl sulfate as a surfactant.

In one embodiment, the powder may include 15 wt% to 55 wt% of an active ingredient, 5 wt% to 24 wt% of lactose hydrate, 7 wt% to 30 wt% of sucrose, 15 wt% to 30 wt% of crospovidone, 1.5 wt% to 5 wt% of polyvinylpyrrolidone, and 0.5 wt% to 10 wt% of sodium lauryl sulfate.

In another embodiment, the powder may include 35 wt% to 55 wt% of an active ingredient, 5 wt% to 24 wt% of lactose hydrate, 7 wt% to 30 wt% of sucrose, 15 wt% to 30 wt% of crospovidone, 1.5 wt% to 5 wt% of polyvinylpyrrolidone, and 0.2 wt% to 1.5 wt% of sodium lauryl sulfate. The powder is advantageous in that the powder includes a high percentage of the active ingredient with respect to the total weight of the preparation and may exhibit an equivalent dissolution rate to that of currently commercially available deferasirox dispersible tablets, thus exhibiting equivalent efficacy to efficacy thereof, and includes a lower percentage of additives included in the preparation than that in currently commercially available deferasirox dispersible tablets, thus reducing side effects due to the additives. In addition, the powder may include a small amount of sodium lauryl sulfate, and thus includes a small amount of surfactant and, accordingly, concern on side effects due to the surfactant may be reduced.

The powder may contain a daily dose required for the treatment of hemochromatosis or iron overload of transfusion-dependent anemias per unit dosage form. The daily dose may vary according to specific diseases, age, gender, body weight, and the like, and may range from, for example, 5 mg/kg (body weight) to 40 mg/kg (body weight), more particularly, about 30 mg/kg.

In one embodiment, the powder may include free acid as an active ingredient in an amount of about 100 mg to about 1,500 mg, more particularly, about 100 mg to about 1,000 mg, per unit dosage form.

In one embodiment, the powder includes the active ingredient in the form of free acid in an amount of about 125 mg, about 250 mg, about 500 mg, or about 1,000 mg per unit dosage form.

The powder includes relatively small amounts of additives compared to those in conventional commercially available dispersible tablets, and may include the active ingredient in an amount of up to 1,000 mg per unit dosage form, due to pharmaceutical properties of powders themselves that have no difficulty in intake unlike big tablets. For example, patients having a body weight of 35 kg or more should ingest 1,000 mg or more of an active ingredient, and thus should take two Exjade 500 mg tablets as commercially available dispersible tablets, but it is advantageous in that the powder according to the present invention may be administered only as a single unit dosage form.

The powder may be directly orally administered or administered as powder for suspension which is suspended in an aqueous phase (e.g., water) to be administered.

Another embodiment of the present invention provides
a method of preparing powder, including: weighing raw materials including an active ingredient, lactose hydrate, and sucrose; and
mixing, kneading, granulating, and milling the weighed raw materials.

The detailed description of the powder according to an embodiment of the present invention may be directly applied to the detailed description of the method of preparing powder.

A method of preparing powder using a high speed mixer, which is known in the art, may be applied to the mixing, kneading, granulating, and milling processes performed using a high speed mixer.

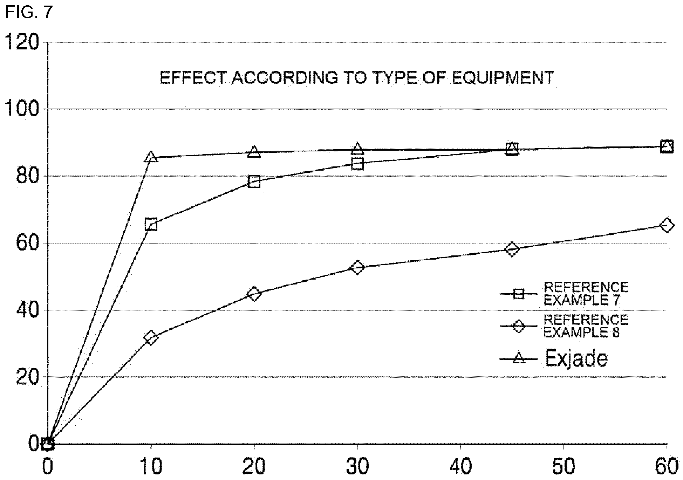

As a result of experimentation, when the mixing, kneading, granulating, and milling processes were performed using a high speed mixer, the dissolution rate of the active ingredient was significantly superior to that in a case in which an extruder was used (see FIG. 7).

### [Examples]

Hereinafter, the present invention will be described in further detail with reference to the following examples. However, these examples are provided for illustrative purposes only and are not intended to limit the scope of the present invention.

### Examples 1 to 6 and Reference Examples 1 to 9: Preparation of Powders according to Various Variables

Deferasirox powders were prepared according to compositions shown in Tables 1 to 3 below.

Components according to each of examples and reference examples shown in Tables 1 to 3 below were weighed on parts-by-weight basis, and then were put in a high speed mixer and subjected to mixing, kneading, granulating, and milling therein, followed by mixing in a drum mixer, thereby completing the preparation of deferasirox powders. Deferasirox powder according to Reference Example 8 was prepared in the same manner as that described above, except that an extruder was used instead of the high speed mixer.

**[Table 1]**

| | Component Name | Ratio of lactose hydrate: MCC | | | Amount of Tween 80 | | |
|---|---|---|---|---|---|---|---|
| | | Reference Example 1 | Reference Example 2 | Reference Example 3 | Reference Example 4 | Reference Example 5 | Reference Example 6 |
| | | | | | | | |
| Main component | Deferasirox | 37.74 | 37.74 | 37.74 | 37.74 | 37.74 | 37.74 |
| Filler | Lactose hydrate | 7.7 | 15.415 | 23.13 | 24.45 | 25.95 | 27.45 |
| | Microcrystalline Cellulose (MCC 101) | 23.13 | 15.415 | 7.7 | 8.15 | 8.65 | 9.15 |
| | Sucrose | | | | | | |
| | Prosolv HD90 | | | | | | |
| Disintegrating agent | Crospovidone | 18.87 | 18.87 | 18.87 | 18.87 | 18.87 | 18.87 |
| Binder | PVP K30 | 3.77 | 3.77 | 3.77 | 3.77 | 3.77 | 3.77 |
| Solubilizer | Sodium lauryl sulfate (SLS) | | | | | | |
| | Tween 80 | 8.79 | 8.79 | 8.79 | 7.02 | 5.02 | 3.02 |
| Total | | 100 | 100 | 100 | 100 | 100 | 100 |

**[Table 2]**

| | Component Name | Type of equipment | | Type of filler | |
|---|---|---|---|---|---|
| | | Reference Example 7 (high speed mixer) | Reference Example 8 (Extruder) | Example 1 | Reference Example 9 |
| Main component | Deferasirox | | 37.74 | 37.74 | 37.74 |
| Filler | Lactose hydrate | 23.13 | 23.13 | 23.13 | 23.13 |
| | Microcrystalline Cellulose (MCC 101) | 7.7 | 7.7 | | |
| | Sucrose | | | 7.7 | |
| | Prosolv HD90 | | | | 7.7 |
| Disintegrating agent | Crospovidone | 18.87 | 18.87 | 18.87 | 18.87 |
| Binder | PVP K30 | 3.77 | 3.77 | 3.77 | 3.77 |
| Solubilizer | Sodium lauryl sulfate (SLS) | 8.79 | 8.79 | 8.79 | 8.79 |
| | Tween 80 | | | | |
| Total | | 100 | 100 | 100 | 100 |

**[Table 3]**

| | Component Name | Ratio of lactose hydrate: sucrose | | Amount of SLS | | |
|---|---|---|---|---|---|---|
| | | Example 2 | Example 3 | Example 4 | Example 5 | Example 6 |
| Main component | Deferasirox | 37.74 | 37.74 | 37.7 | 37.7 | 37.7 |
| Filler | Lactose hydrate | 15.415 | 7.7 | 8.7 | 9.3 | 9.8 |
| | Microcrystalline Cellulose (MCC 101) | | | | | |
| | Sucrose | 15.415 | 23.13 | 26 | 27.9 | 29.4 |
| | Prosolv HD90 | | | | | |
| Disintegrating agent | Crospovidone | 18.87 | 18.87 | 18.8 | 18.8 | 18.8 |
| Binder | PVP K30 | 3.77 | 3.77 | 3.8 | 3.8 | 3.8 |
| Solubilizer | Sodium lauryl sulfate (SLS) | 8.79 | 8.79 | 5 | 2.5 | 0.5 |
| | Tween 80 | | | | | |
| Total | | 100 | 100 | 100 | | 100 |

### Experimental Example 1: Dissolution Evaluation

Dissolution evaluation was performed on each of the deferasirox powders of Examples 1 to 6 and Reference Examples 1 to 9 prepared according to Tables 1 to 3 above. A dissolution test was performed on each powder under the following test conditions, and an average dissolution rate (wt%) was calculated. The results thereof are shown in Tables 4 to 9 below according to the type of filler (Table 4), the ratio of lactose hydrate to sucrose as a filler (Table 5), the ratio of lactose hydrate to microcrystalline cellulose (MCC) as a filler (Table 6), the type of surfactant (Table 7), the amount of Tween 80 (Table 8), the amount of SLS (Table 9), and the type of equipment (Table 10), and graphs of the dissolution rates are illustrated in FIGS. 1 to 6.

### <Dissolution Test Conditions>

Dissolution test method: Dissolution Test Method 2 (Paddle method) in the Korean Pharmacopoeia
Dissolution medium: pH 6.8 medium + 1% Tween 20
Amount of dissolution medium: 900 mL
Temperature of dissolution medium: 37.5 °C
Paddle rate: 50 rpm
Number of test samples: 6
Sample collection time: 10 minutes, 20 minutes, 30 minutes, 45 minutes, and 60 minutes

**[Table 4]**

| Dissolution time | Type of filler | | | Control |
|---|---|---|---|---|
| | Reference Example 7 | Example 1 | Reference Example 9 | Exjade |
| | Lactose + MCC | Lactose + Sucrose | Lactose + Prosolv HD90 | |
| 0 | 0 | 0 | 0 | 0 |
| 10 | 65.81 | 81.27 | 78.87 | 85.64 |
| 20 | 78.78 | 85.58 | 81.83 | 87.37 |
| 30 | 83.91 | 86.59 | 85.41 | 87.93 |
| 45 | 88.24 | 86.23 | 86.49 | 87.75 |
| 60 | 88.73 | 86.52 | 87.76 | 88.86 |

**[Table 5]**

| Dissolution time | Lactose hydrate: Sucrose ratio | | | Control |
|---|---|---|---|---|
| | Example 1 (1:3) | Example 2 (1:1) | Example 3 (3:1) | Exjade |
| 0 | 0 | 0 | 0 | 0 |
| 10 | 81.27 | 81.23 | 81.34 | 85.64 |
| 20 | 85.58 | 82.67 | 83.17 | 87.37 |
| 30 | 86.59 | 83.8 | 88.18 | 87.93 |
| 45 | 86.23 | 84.33 | 84.72 | 87.75 |
| 60 | 86.52 | 85.19 | 87.41 | 88.86 |

**[Table 6]**

| Dissolution time | Lactose hydrate: MCC ratio | | | Control |
|---|---|---|---|---|
| | Reference Example 1 (1:3) | Reference Example 2 (1:1) | Reference Example 3 (3:1) | Exjade |
| 0 | 0 | 0 | 0 | 0 |
| 10 | 46.16 | 50.47 | 55.61 | 85.64 |
| 20 | 67.4 | 68.44 | 69.97 | 87.37 |
| 30 | 77.69 | 78.31 | 78.03 | 87.93 |
| 45 | 78.97 | 79.9 | 80.6 | 87.75 |
| 60 | 79.38 | 79.56 | 81.49 | 88.86 |

**[Table 7]**

| Dissolution time | Type of surfactant | | Control |
|---|---|---|---|
| | Reference Example 3 | Reference Example 7 | Exjade |
| | Tween | SLS | |
| 0 | 0 | 0 | 0 |
| 10 | 55.61 | 65.81 | 85.64 |
| 20 | 69.97 | 78.78 | 87.37 |
| 30 | 78.03 | 83.91 | 87.93 |
| 45 | 80.6 | 88.24 | 87.75 |
| 60 | 81.49 | 88.73 | 88.86 |

**[Table 8]**

| Dissolution time | Amount of Tween 80 | | | | Control |
|---|---|---|---|---|---|
| | Reference Example 3 (8.79%) | Reference Example 4 (7.02%) | Reference Example 5 (5.02%) | Reference Example 6 (3.02%) | Exjade |
| 0 | 0 | 0 | 0 | 0 | 0 |
| 10 | 55.61 | 50.58 | 47.7 | 42.78 | 85.64 |
| 20 | 69.97 | 65.69 | 65.54 | 60.92 | 87.37 |
| 30 | 78.03 | 77.92 | 78.357 | 78.88 | 87.93 |
| 45 | 80.6 | 82.08 | 81.01 | 81.43 | 87.75 |
| 60 | 81.49 | 80.16 | 80.28 | 80.78 | 88.86 |

**[Table 9]**

| Dissolution time | Amount of SLS | | | | Control |
|---|---|---|---|---|---|
| | Example 1 (8.79%) | Example 4 (5%) | Example 5 (2.5%) | Example 6 (0.5%) | Exjade |
| 0 | 0 | 0 | 0 | 0 | 0 |
| 10 | 81.27 | 83.24 | 83.95 | 84.26 | 85.64 |
| 20 | 85.58 | 85.25 | 86.8 | 86.67 | 87.37 |
| 30 | 86.59 | 86.82 | 86.32 | 86.26 | 87.93 |
| 45 | 86.23 | 86.33 | 85.51 | 87.48 | 87.75 |
| 60 | 86.52 | 86.19 | 87.3 | 87.92 | 88.86 |

**[Table 10]**

| Dissolution time | Type of equipment | | Control |
|---|---|---|---|
| | Reference Example 7 (high speed mixer) | Reference Example 8 (extruder) | Exjade |
| 0 | 0 | 0 | 0 |
| 10 | 65.81 | 32.24 | 85.64 |
| 20 | 78.78 | 44.91 | 87.37 |
| 30 | 83.91 | 52.94 | 87.93 |
| 45 | 88.24 | 58.21 | 87.75 |
| 60 | 88.73 | 65.65 | 88.86 |

According to FIG. 1 and Table 4, among the fillers, the combination of lactose and sucrose exhibited the highest dissolution rate compared to the combination of lactose and microcrystalline cellulose (MCC) and the combination of lactose and silicified microcrystalline cellulose (Prosolv HD90), and exhibited an almost equivalent dissolution rate to that of Exjade.

According to FIG. 2 and Table 5, there was seen to be nearly no difference between dissolution rates according to ratios of lactose to sucrose.

According to FIG. 3 and Table 6, it was shown that cases of various ratios of lactose to microcrystalline cellulose did not significantly reach the dissolution rate of Exjade.

According to FIG. 4 and Table 7, it was shown that the powder including sodium lauryl sulfate (SLS) as a surfactant exhibited a significantly high dissolution rate compared to the case of a Tween 80-containing powder.

According to FIG. 5 and Table 8, it was shown that cases of various percentages of Tween 80 did not significantly reach the dissolution rate of Exjade.

According to FIG. 6 and Table 9, it was shown that there was nearly no difference between dissolution rates according to the percentage of SLS.

According to FIG. 7 and Table 10, a case in which the powder was prepared using a high speed mixer exhibited a significantly high dissolution rate as compared to a case in which the powder was prepared using an extruder.

The present invention has been described with reference to example embodiments thereof. It will be understood by one of ordinary skill in the art to which the present invention pertains that the invention may be embodied in various modified forms without departing from the essential characteristics thereof. Thus, the embodiments described herein should be considered in an illustrative sense only and not for the purpose of limitation. The scope of the present invention is defined by the following claims rather than the foregoing description, and all changes or modified forms equivalent thereto should be construed as being within the scope of the present invention.

## Claims

1. Powder comprising 20 wt% to 45 wt% of a combination of lactose and sucrose with respect to a total weight of the powder, and including, as an active ingredient, a compound represented by Formula 1 below or a pharmaceutically acceptable salt thereof:

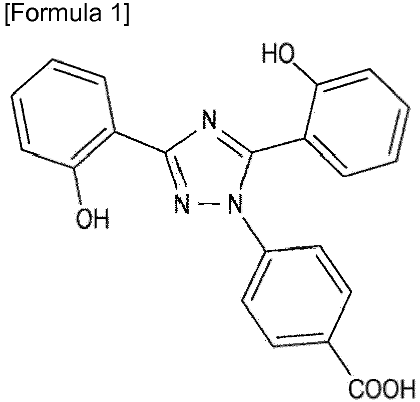

2. The powder of claim 1, wherein the combination of lactose and sucrose is included in an amount of 25 wt% to 42 wt%.

3. The powder of claim 1, wherein the combination of lactose and sucrose is included in a weight ratio of 1:1 to 1:4.

4. The powder of claim 3, wherein the combination of lactose and sucrose is included in a weight ratio of 1:2.5 to 1:3.5.

5. The powder of claim 1, further comprising a pharmaceutical additive selected from the group consisting of a disintegrating agent, a binder, a surfactant, and combinations thereof.

6. The powder of claim 1, wherein the disintegrating agent is selected from the group consisting of crospovidone, croscarmellose sodium, sodium starch glycolate, low substituted hydroxypropylcellulose, and combinations thereof,
the binder is selected from the group consisting of polyvinylpyrrolidone, hydroxypropylcellulose, hydroxypropylmethylcellulose, light anhydrous silicic acid, aluminum silicate, calcium silicate, calcium hydrogen phosphate, calcium carbonate, and combinations thereof, and
the surfactant is selected from the group consisting of sodium lauryl sulfate, polyoxyethylene fatty acid esters, and combinations thereof.

7. The powder of claim 6, wherein the surfactant is sodium lauryl sulfate.

8. The powder of claim 7, wherein the sodium lauryl sulfate is included in an amount of 0.2 wt% to 0.5 wt% or less with respect to the total weight of the powder.

9. The powder of claim 5, wherein the disintegrating agent is crospovidone, the binder is polyvinylpyrrolidone, and the surfactant is sodium lauryl sulfate.

10. The powder of claim 1, wherein the powder comprises 35 wt% to 55 wt% of the active ingredient, 5 wt% to 24 wt% of lactose hydrate, 7 wt% to 30 wt% of sucrose, 15 wt% to 30 wt% of crospovidone, 1.5 wt% to 5 wt% of polyvinylpyrrolidone, and 0.2 wt% to 1.5 wt% of sodium lauryl sulfate.

11. The powder of claim 1, wherein the active ingredient is included in an amount of 35 wt% to 50 wt% with respect to the total weight of the powder.

12. The powder of claim 1, wherein the powder includes, as the active ingredient, a pharmaceutically effective amount of an iron chelator per unit dosage form.

13. The powder of claim 1, wherein the powder includes free acid as the active ingredient in an amount of 100 mg to 1,000 mg.

14. The powder of claim 1, wherein the powder is directly administered or administered as a suspension.

15. A method of preparing the powder according to any one of claims 1 to 14, the method comprising:
weighing raw materials comprising an active ingredient, lactose, and sucrose; and
mixing, kneading, granulating, and milling the weighed raw materials.

16. The method of claim 15, wherein the mixing, kneading, granulating, and milling are performed using a high speed mixer.

## Amended claims

### Amended claims under Art. 19.1 PCT

1. Powder comprising 20 wt% to 45 wt% of a combination of lactose and sucrose with respect to a total weight of the powder, and including, as an active ingredient, a compound represented by Formula 1 below or a pharmaceutically acceptable salt thereof:

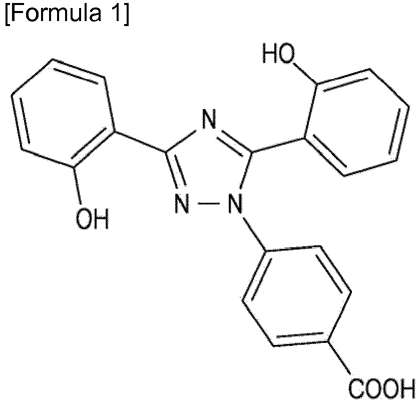

2. The powder of claim 1, wherein the combination of lactose and sucrose is included in an amount of 25 wt% to 42 wt%.

3. The powder of claim 1, wherein the combination of lactose and sucrose is included in a weight ratio of 1:1 to 1:4.

4. The powder of claim 3, wherein the combination of lactose and sucrose is included in a weight ratio of 1:2.5 to 1:3.5.

5. The powder of claim 1, further comprising a pharmaceutical additive selected from the group consisting of a disintegrating agent, a binder, a surfactant, and combinations thereof.

6. (Amended) The powder of claim5, wherein the disintegrating agent is selected from the group consisting of crospovidone, croscarmellose sodium, sodium starch glycolate, low substituted hydroxypropylcellulose, and combinations thereof,
the binder is selected from the group consisting of polyvinylpyrrolidone, hydroxypropylcellulose, hydroxypropylmethylcellulose, light anhydrous silicic acid, aluminum silicate, calcium silicate, calcium hydrogen phosphate, calcium carbonate, and combinations thereof, and
the surfactant is selected from the group consisting of sodium lauryl sulfate, polyoxyethylene fatty acid esters, and combinations thereof.

7. The powder of claim 6, wherein the surfactant is sodium lauryl sulfate.

8. (Amended) The powder of claim 7, wherein the sodium lauryl sulfate is included in an amount of 0.2 wt% to 0.5 wt% with respect to the total weight of the powder.

9. The powder of claim 5, wherein the disintegrating agent is crospovidone, the binder is polyvinylpyrrolidone, and the surfactant is sodium lauryl sulfate.

10. (Amended)The powder of claim9, wherein the powder comprises 35 wt% to 55 wt% of the active ingredient, 5 wt% to 24 wt% of lactose hydrate, 7 wt% to 30 wt% of sucrose, 15 wt% to 30 wt% of crospovidone, 1.5 wt% to 5 wt% of polyvinylpyrrolidone, and 0.2 wt% to 1.5 wt% of sodium lauryl sulfate.

11. The powder of claim 1, wherein the active ingredient is included in an amount of 35 wt% to 50 wt% with respect to the total weight of the powder.

12. The powder of claim 1, wherein the powder includes, as the active ingredient, a pharmaceutically effective amount of an iron chelator per unit dosage form.

13. The powder of claim 1, wherein the powder includes free acid as the active ingredient in an amount of 100 mg to 1,000 mg.

14. The powder of claim 1, wherein the powder is directly administered or administered as a suspension.

15. A method of preparing the powder according to any one of claims 1 to 14, the method comprising:
weighing raw materials comprising an active ingredient, lactose, and sucrose; and
mixing, kneading, granulating, and milling the weighed raw materials.

16. The method of claim 15, wherein the mixing, kneading, granulating, and milling are performed using a high speed mixer.
